# EUROPEAN PATENT APPLICATION

(11) **EP 2 868 340 A1**
(43) Date of publication of application: **06.05.2015**
(21) Application number: 14191453.1
(22) Date of filing: 03.11.2014
(51) Int. Cl.: A61M 25/01

(54) **Positioning catheter**

(30) Priority: 01.11.2013 US 201361898691 P; 30.07.2014 US 201414447274
(71) Applicant: Covidien LP, Mansfield, MA 02048 (US)
(72) Inventor: Costello, David M., Delano, Minnesota 55328 (US); Crowley, Thomas P., Lino Lakes, Minnesota 55014 (US)
(74) Representative: Hoffmann Eitle

(57) **Abstract**

A catheter guide assembly includes an extended working channel having a proximal portion adjacent a proximal end and a distal portion adjacent a distal end, the extended working channel having a lumen configured to enable translation of an instrument therethrough. The extended working channel defines a radius formed on the distal portion, wherein the distal portion of the extended working channel is formed of a material having a durometer rating sufficient such that deflection of the distal portion is limited as the instrument is translated therethrough. The catheter guide assembly includes a control handle disposed at the proximal end of the extended working channel and operably coupled to the extended working channel, thereby enabling the extended working channel to advance and rotate.

## Description

### CLAIM OF PRIORITY

The present application claims priority to earlier filed U.S. Provisional Patent Application No. 61/898,691 entitled EDGE EWC Catheter Tip Deflection filed November 1, 2013, the entire contents of which are incorporated herein by reference.

### BACKGROUND

### Technical Field

The present disclosure relates to a positioning catheter. More particularly, the present disclosure relates to a positioning catheter including a distal portion adjacent a distal end with a durometer rating sufficient such that deflection of the distal portion is limited when a surgical instrument is inserted into the positioning catheter.

### Description of Related Art

Microwave ablation systems may be utilized for treating various maladies, e.g., potentially cancerous growths on different organs, such as, for example, the liver, brain, heart, lung, and kidney. A microwave ablation system may include a microwave energy source, an ablation probe, and one or more guide members, e.g., a positioning catheter, that are used to guide a microwave ablation probe adjacent target tissue. The positioning catheter, as well as the microwave ablation probe, is typically relatively thin and flexible to allow a user to navigate the positioning catheter through a luminal network of an organ, e.g., a lung. In certain instances, a sensor or navigation catheter may be positioned within the positioning catheter to facilitate positioning a distal end of the positioning catheter adjacent target tissue. A location of the distal end of the positioning catheter is then recorded for treatment of the target tissue.

In certain systems, once the location is obtained, the navigation catheter may be removed from the positioning catheter and one or more surgical instruments (e.g., biopsy forceps, cytology brush, aspirating needles, ablation catheters, etc.) may be inserted through the positioning catheter and positioned at or extended from the distal end of the positioning catheter to treat tissue. However, because of the relatively thin and flexible construction of the positioning catheter, extending or positioning the surgical instruments from or at the distal end of the positioning catheter to treat target tissue may cause the distal end of the positioning catheter to deflect from its original position and move relative to the target tissue.

While the existing positioning catheters are suitable for their intended purposes, movement of the distal end of the positioning catheter (e.g., as a result of the surgical instrument being extended from or positioned at the distal end) after the location has been determined, can make it difficult for a clinician to maintain tissue target trajectory. As can be appreciated, this, in turn, may decrease target tissue treatment efficacy.

### SUMMARY

As can be appreciated, a catheter guide assembly including an extended working channel having a distal portion adjacent a distal end with a durometer sufficient such that deflection of the distal portion is limited when a surgical instrument is inserted into the extended working channel may prove useful in the surgical arena.

According to one embodiment of the present disclosure, a catheter guide assembly is provided. The catheter guide assembly includes an extended working channel and a control handle. The extended working channel includes a proximal portion adjacent a proximal end and a distal portion adjacent a distal end, the extended working channel further includes a lumen configured to enable translation of an instrument therein. The extended working channel defines a radius formed on the distal portion, wherein the distal portion of the extended working channel is formed of a material having a durometer rating sufficient such that deflection of the distal portion is limited as the instrument is translated therethrough. The control handle is disposed at the proximal end of the extended working channel and is operably coupled to the extended working channel, thereby enabling the extended working channel to advance and rotate.

In one aspect, the extended working channel is configured for positioning within a bronchoscope. A combination of the catheter guide assembly and the bronchoscope may be provided.

In one aspect, a locatable guide including at least one locatable guide sensor is positionable within the lumen such that the location of the locatable guide within a luminal network can be detected. A combination of the locatable guide and the catheter guide assembly may be provided.

In one aspect, the proximal portion of the extended working channel comprises a first durometer rating and the distal portion of the extended working channel comprises a second durometer rating, wherein the first durometer rating is greater than the second durometer rating.

In one aspect, the durometer rating of the distal end is different from the second durometer rating of the distal portion.

In one aspect, the distal end comprises the first durometer rating.

In one aspect, the distal portion comprises a length that ranges from about 5 percent to about 15 percent of the overall length of the extended working channel.

In one aspect, the first durometer rating ranges from about 30D to about 80D and the second durometer rating ranges from about 30D to about 63D.

According to one embodiment of the present disclosure, a system for accessing a target within a luminal network is provided. The system includes a surgical instrument, an extended working channel, a control handle, a locatable guide, a non-transitory computer readable medium, and a user interface. The extended working channel includes a proximal portion adjacent a proximal end and a distal portion adjacent a distal end, the extended working channel further includes a lumen configured to enable translation of the instrument therein. The extended working channel defines a radius formed on the distal portion, wherein the distal portion of the extended working channel is formed of a material having a durometer rating sufficient such that deflection of the distal portion is limited as the instrument is translated therethrough. The control handle is disposed at the proximal end of the extended working channel and is operably coupled to the extended working channel, thereby enabling the extended working channel to advance and rotate. The locatable guide is insertable through the extended working channel. The non-transitory computer readable medium is configured for storing a program for causing a computer to determine a pathway through a luminal network from an entry point to a target tissue and assign a plurality of waypoints along the pathway. The user interface is configured to display an orientation of the locatable guide relative to the determined pathway as the extended working channel and the locatable guide are translated through the luminal network from the entry point towards the target tissue.

In one aspect, the locatable guide includes at least one locatable guide sensor positionable within the lumen of the extended working channel, wherein the at least one locatable guide sensor is configured to enable detection of a location of the locatable guide within the luminal network of a patient's airways.

In one aspect, the at least one locatable guide sensor is disposed within the lumen of the extended working channel towards the distal portion thereof.

In one aspect, the instrument is selected from the group consisting of a biopsy forceps, a cytology brush, an ablation catheter, and an aspirating needle.

In one aspect, further comprising a tracking system including a tracking module, a plurality of reference sensors, and a transmitter mat, at least one of the plurality of reference sensors being attached to a patient, wherein the transmitter mat generates an electromagnetic field such that the tracking module may be used to determine a location of the at least one of the plurality of reference sensors and the locatable guide sensor.

### BRIEF DESCRIPTION OF THE DRAWINGS

Various embodiments of the present disclosure are now described with references to the drawings, wherein:
Fig. 1 depicts an electromagnetic navigation bronchoscopy system in accordance with the instant disclosure;
Figs. 2A-2B are perspective views of a plurality of surgical instruments in accordance with the present disclosure
Fig. 3 is a side view of the positioning catheter shown in Fig. 1;
Fig. 4A is a side view of the positioning catheter having a radius of curvature of about 45 degrees;
Fig. 4B is a side view of the positioning catheter having a radius of curvature of about 90 degrees;
Fig. 4C is a side view of the positioning catheter having a radius of curvature of about 180 degrees;
Fig. 5A is a side view of the positioning catheter having a radius of curvature of about 45 degrees without an instrument disposed therein;
Fig. 5B is a side view of the positioning catheter having a radius of curvature of about 45 degrees with an instrument disposed therein;
Fig. 6A is a side view of the positioning catheter having a radius of curvature of about 90 degrees without an instrument disposed therein;
Fig. 6B is a side view of the positioning catheter having a radius of curvature of about 90 degrees with an instrument disposed therein;
Fig. 7A is a side view of the positioning catheter having a radius of curvature of about 180 degrees without an instrument disposed therein;
Fig. 7B is a side view of the positioning catheter having a radius of curvature of about 180 degrees with an instrument disposed therein;
Figs. 8A-8E are side views of the positioning catheter having a radius of curvature of about 45 degrees with various instruments disposed therein;
Figs. 9A-9E are side views of the positioning catheter having a radius of curvature of about 90 degrees with various instruments disposed therein;
Figs. 10A-10E are side views of the positioning catheter having a radius of curvature of about 180 degrees with various instruments disposed therein;
Fig. 11A is a schematic, plan view of a bronchoscope positioned within the lungs of a patient with a positioning catheter extending distally therefrom; and
Fig. 11B is an enlarged area of detail of Fig. 11A, which shows bifurcations in the luminal network of a patient's airways and the positioning catheter located therein.

### DETAILED DESCRIPTION

The present disclosure is directed in part to a positioning catheter or extendable working channel including a distal portion with a durometer rating sufficient such that deflection of the distal portion is limited when a surgical instrument is inserted into the positioning catheter and positioned at the distal end.

Detailed embodiments of the present disclosure are disclosed herein; however, the disclosed embodiments are merely examples of the disclosure, which may be embodied in various forms. Therefore, specific structural and functional details disclosed herein are not to be interpreted as limiting, but merely as a basis for the claims and as a representative basis for teaching one skilled in the art to variously employ the present disclosure in virtually any appropriately detailed structure.

Aspects of the present disclosure are described in detail with reference to the drawing figures wherein like reference numerals identify similar or identical elements. As used herein, the term "distal" refers to the portion that is being described which is further from a user, while the term "proximal" refers to the portion that is being described which is closer to a user.

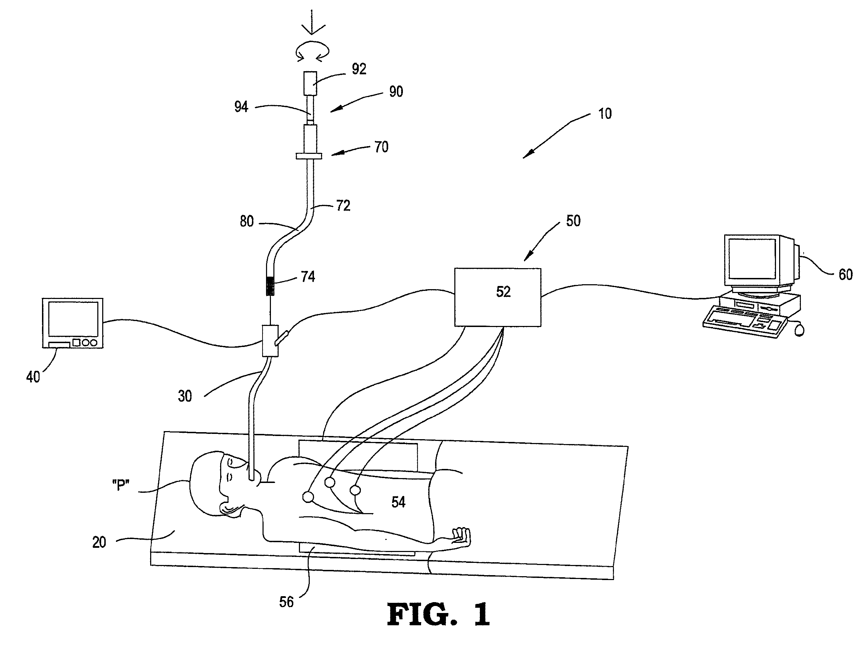

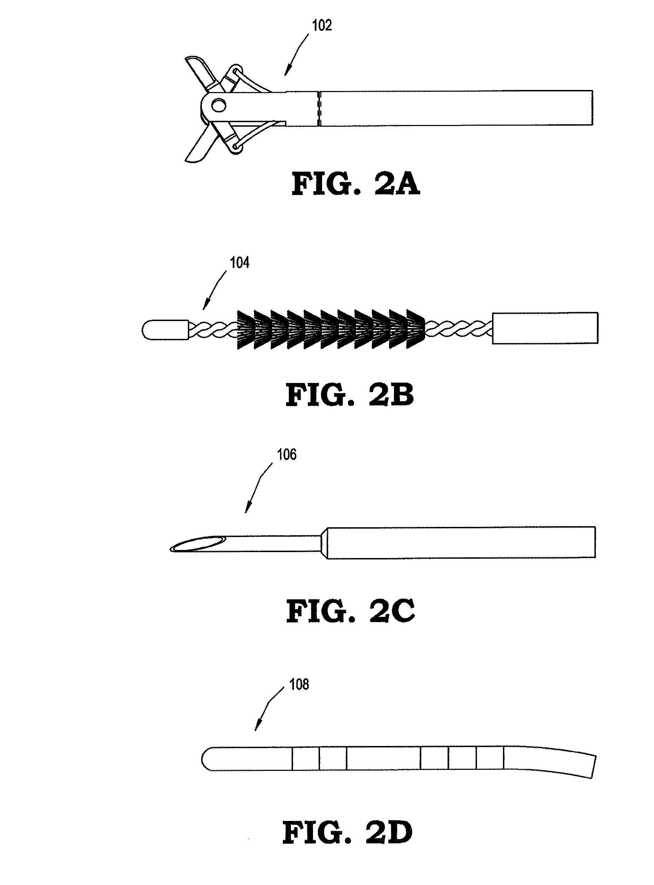

Fig. 1 depicts an electromagnetic navigation bronchoscopy (ENB) system 10 in accordance with the present disclosure. The ENB system 10 is configured for planning a pathway to a target tissue 68 (planning phase) and navigating an extended working channel 80 to the target tissue 68 (navigation phase). Following navigation, surgical instruments such as those depicted in Figs. 2A-2D, including for example, a biopsy forceps 102 (Fig. 2A), a cytology brush 104 (Fig. 2B), an aspirating needle 106 (Fig. 2C), and an ablation catheter 108 (Fig. 2D) may be inserted into the extended working channel 80 to obtain a tissue sample from the target tissue 68. The ENB system 10 generally includes an operating table 20 configured to support a patient "P"; a bronchoscope 30 configured for insertion through the patient "P's" mouth and into the patient "P's" airways; a monitoring equipment 40 coupled to the bronchoscope 30 for displaying video images received from the bronchoscope 30; a tracking system 50 including a tracking module 52, a plurality of reference sensors 54, and a transmitter mat 56; a computer 60 including software and/or hardware used to facilitate pathway planning, identification of the target tissue 68, and navigation to the target tissue 68; a catheter guide assembly 70 including extended working channel 80, a locatable guide 72 insertable into extended working channel 80 and having a sensor 74 at a distal end 73; and a steering assembly, such as, for example, a control handle 90.

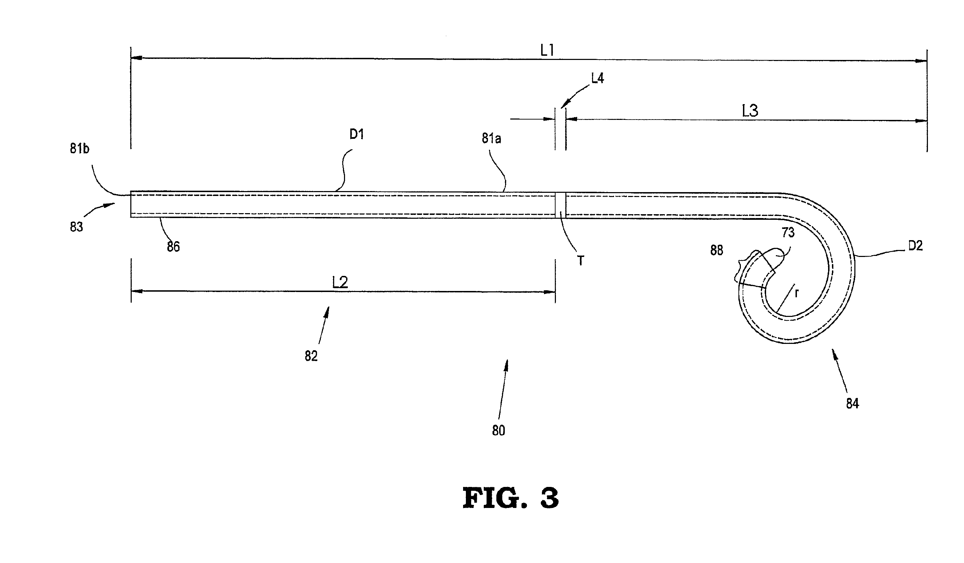

Fig. 3 is a perspective view of extended working channel 80 according to an embodiment of the present disclosure. Extended working channel 80 extends longitudinally between a proximal portion 82 and a distal portion 84. Extended working channel 80 includes a proximal end 86 adjacent proximal portion 82 and a distal end 88 adjacent distal portion 84. In embodiments, distal portion 84 of extended working channel 80 may be shaped to define at-rest, a linear, a curved, or an angled configuration, depending on the particular purpose of the extended working channel 80.

Extended working channel 80 further includes an outer surface 81 a and an inner surface 81 b defining a lumen 83 therein. In one embodiment, the surfaces 81a and 81 b may include various tribological properties, including, but not limited to, decreased coefficient of friction and increased lubrication. As shown in Fig. 3, lumen 83 is sized to receive locatable guide 72 or surgical instruments 102-108.

The extended working channel 80 may be formed from any suitable material, including but not limited to, rubbers and plastics acceptable for surgical and medical use. It is contemplated that a suitable material (not specifically shown) may include a braided support structure formed from metals or alternatively one or more non-conductive fibrous materials such as Kevlar or other aramid fibers to provide additional resilience and to maintain lumen 83 in a generally open configuration to ease the passage of locatable guide 72, surgical instruments 102-108, and other tools therethrough.

As shown in Fig. 3, the extended working channel 80 defines a length "L1". In one embodiment, the extended working channel 80 may be formed from materials having a uniform durometer rating along the length "L1". In some embodiments, the proximal portion 82 may be formed from materials having a durometer rating "D1" ranging from about 30D to about 80D along a length "L2" of the extended working channel 80. However, durometer rating "D1" of proximal portion 82 is not limited to the range specified above, and may be greater or less. In another embodiment, distal portion 84 may be formed from materials having a durometer rating "D2" ranging from about 30D to about 63D along a length "L3". However, it is also contemplated that durometer rating "D2" of distal portion 84 may be greater or less than these specified ranges.

When proximal portion 82 has the durometer rating "D1" and distal portion 84 has the durometer rating "D2", durometer rating "D1" is typically greater than durometer rating "D2". However, in some embodiments, durometer rating "D1" may be less than durometer rating "D2". In some embodiments, proximal portion 82 and distal end 88 may be composed of the same material. In such an embodiment, the proximal portion 82 and the distal end 88 may have the same durometer rating, such as, for example, durometer rating "D1", which may be greater than the durometer rating "D2" of distal portion 84. However, in alternative embodiments, distal end 88 may be composed of the same material as distal portion 84 and therefore have the same durometer rating.

It is contemplated that increasing the durometer rating "D2" will limit the deflection of distal portion 84 when one or more of surgical instruments 102-108 or locatable guide 72 is disposed within lumen 83 of extended working channel 80. The limited deflection will in turn improve the ability of the user to maintain a target trajectory within the patient "P's" airways following the removal of locatable guide 72 or surgical instruments 102-108.

In the ENB system 10 of Fig. 1, during insertion and navigation of extended working channel 80 and locatable guide 72 to a desired target, the location of the extended working channel 80 is determined with the help of locatable guide 72, and more particularly, the sensor 74 affixed to distal end 73 of locatable guide 72. In one embodiment, in order to place surgical instruments 102-108 into lumen 83 of extended working channel 80, locatable guide 72 including sensor 74 may need to be removed. The user will typically use fluoroscopy to visualize any motion of extended working channel 80, however, it is very useful to the user for positioning purposes to be able to anticipate movement of the extended working channel 80 upon removal/placement of the surgical instruments 102-108 within lumen 83.

Accordingly, by employing extended working channel 80 having specific mechanical properties (e.g., durometer ratings) according to the present disclosure, the effect of withdrawing locatable guide 72 and the subsequent insertion of surgical tools 102-108 can be ascertained with a greater degree of accuracy. In particular, before removing locatable guide 72, the user is able to properly place the extended working channel 80 within the luminal network of patient "P" to accommodate for the expected effect of inserting surgical instruments 102-108. Moreover, because each surgical instrument 102-108 has a different effect on the shape of extended working channel 80 when inserted within lumen 83, each of these may be separately ascertained and understood by the user.

In embodiments where the durometer rating of the extended working channel 80 changes between the proximal portion 82 and the distal portion 84, the extended working channel 80 includes a transition portion "T", as shown in Fig. 3. The transition portion "T" may be formed from suitable connection methods, including, but no limited to, over molding, thermal fusion, and ultrasonic welding. The distance between the proximal end 86 and the transition portion "T" is length "L2". In one embodiment, length "L2" ranges from about 900mm to about 1000mm. The distance between distal end 88 and transition portion "T" is length "L3". In one embodiment, length "L3" ranges from about 90mm to about 100mm. As shown in Fig. 3, the length of transition portion "T" is a length "L4". In one embodiment, length "L4" is approximately 10mm.

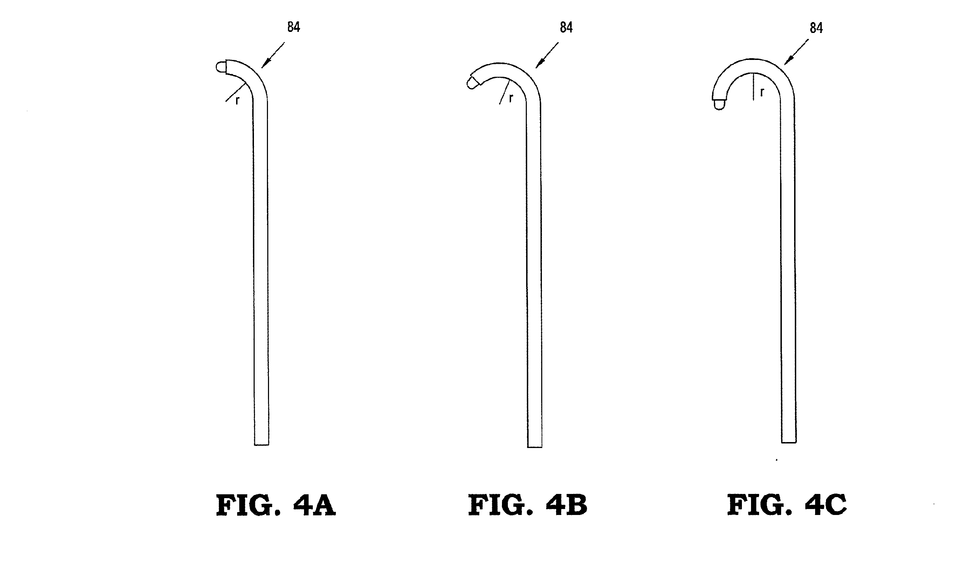

With continued reference to Figs. 3 and Fig. 4A-4C, the distal portion 84 may have a curved or hooked configuration. Differing amounts of pre-curved implementations in the extended working channel 80 may be used to navigate to differing portions of the patient "P's" airways. For example, in one embodiment, the extended working channel 80 including a radius of curvature "r" of 180 degrees may be used for directing locatable guide 72 and to a posterior portion of the upper lobe of the patient "P's" airways. Common radii of curvatures "r", include 45 (Fig. 4A), 90 (Fig. 4B), and 180 degrees (Fig. 4C), however, other radii of curvatures "r" may be employed without departing from the scope of the present disclosure.

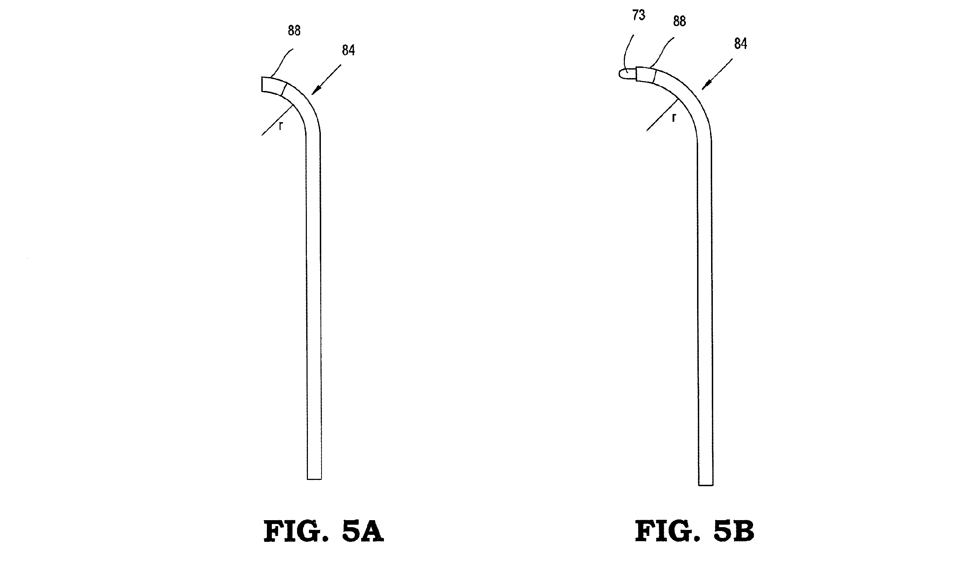

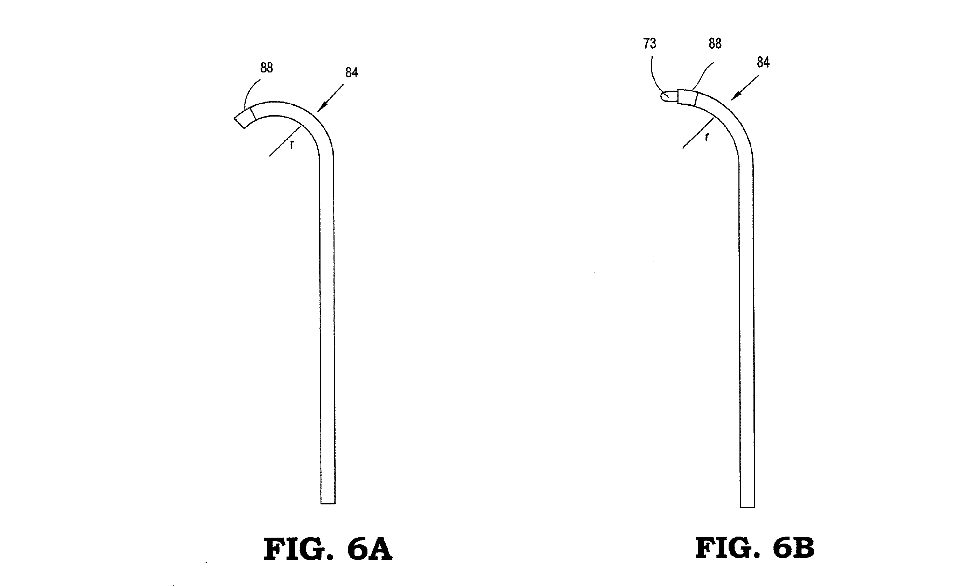

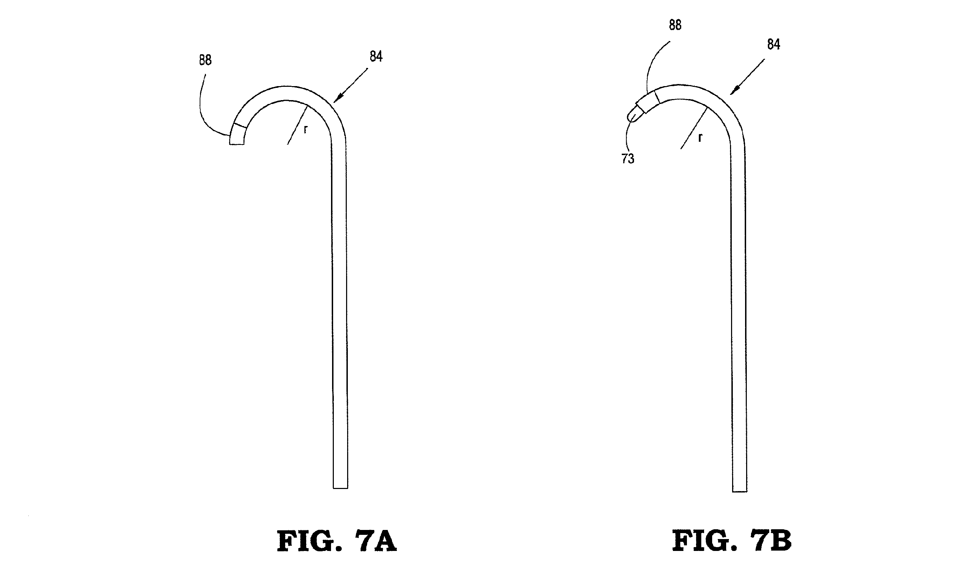

Fig. 5A is a side view of an embodiment of distal portion 84 of the extended working channel 80. In this embodiment, distal portion 84 has the radius of curvature "r" of about 45 degrees. In accordance with the instant disclosure, when locatable guide 72 (Fig. 5B) is disposed in the extended working channel 80 such that sensor 74 is extended past distal end 88, the radius of curvature "r" (or deflection shape) of distal portion 84 remains substantially unchanged (compare Fig. 5A with Fig. 5B). Similar results are attained when distal portion 84 has the radius of curvature "r" of about 90 degrees or the radius of curvature "r" of about 180 degrees (see Figs. 6A, 6B and Figs. 7A, 7B, respectively).

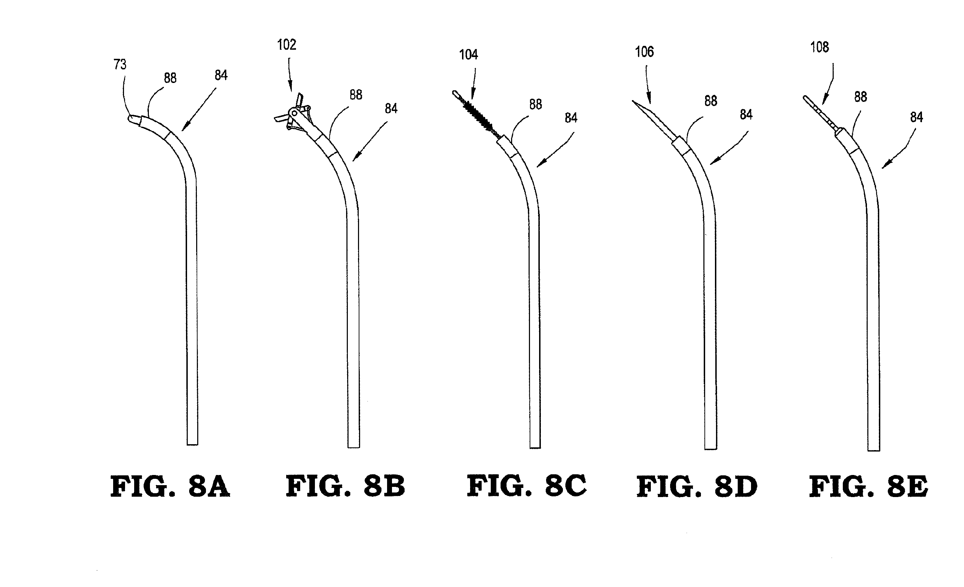

Figs. 8A-8E illustrate various surgical instruments extending from distal end 88 of the distal portion 84, which initially has the radius of curvature "r" of about 45 degrees. Specifically, Figs. 8A, 8B, 8C, 8D, and 8E show locatable guide 72, biopsy forceps 102, cytology brush 104, aspirating needle 106, and ablation catheter 108, respectively, extending from distal end 88 of the distal portion 84. As can be seen from Figs. 8A-E, theses various surgical instruments 102-108 can be disposed within lumen 83 of extended working channel 80 and translated past the distal end 88 to treat the target tissue 68 while substantially maintaining the about 45 degree radius of curvature "r" of distal portion 84 (compare Figs. 8A-8E with Figs. 4A-C). Moreover, it is contemplated that where there is a change in the radius of curvature "r" of distal portion 84, the amount of change can be predicted for a specific instrument such that the expected change in the radius of curvature "r" can be accounted for by the user in placing the extended working channel 80 and before removing locatable guide 72.

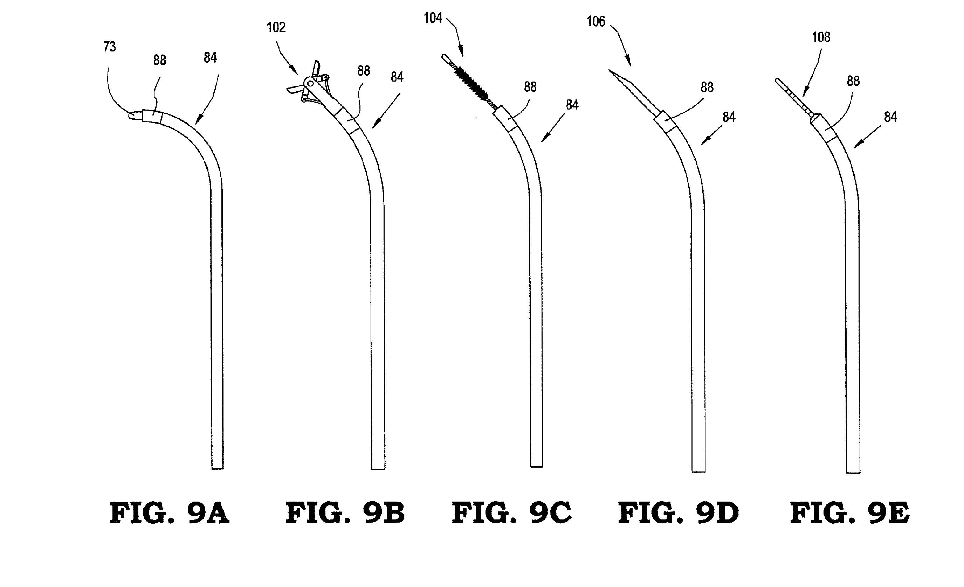

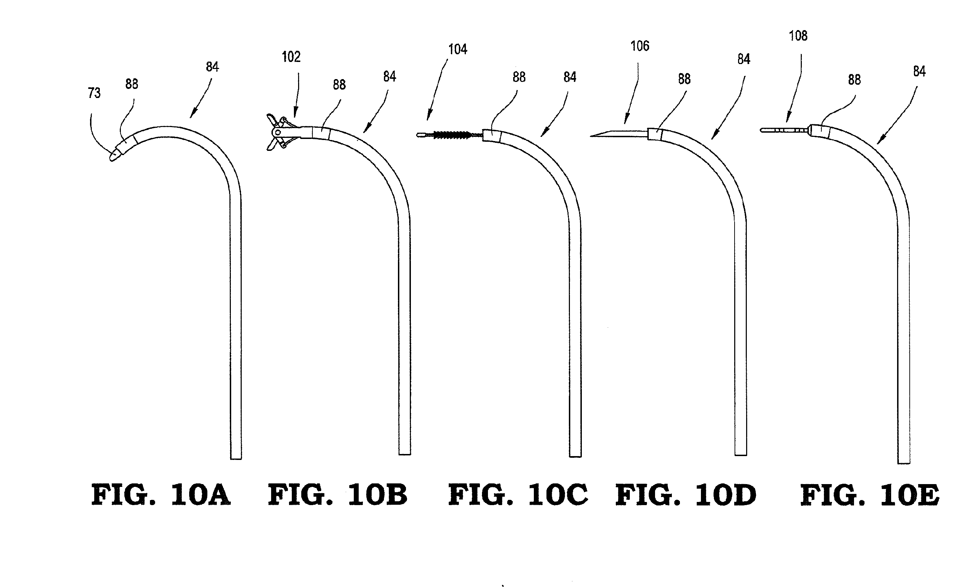

The same or similar results are obtained when distal portion 84 is provided with the radius of curvature "r" of about 90 degrees or the radius of curvature "r" of about 180 degrees (see Figs. 9A-9E and 10A-10E, respectively).

Figs. 8A, 9A, and 10A depict the radius of curvature "r" of extended working channel 80 when locatable guide 72 is disposed within lumen 83 of extended working channel 80. Figs. 8B-E, 9B-E, and 10B-E depict the deflection of distal portion 84 of extended working channel 80 when locatable guide 72 is removed from lumen 83 and replaced by surgical instruments 102-108. The deflection of the distal portion 84 when surgical instruments 102-108 are disposed within the extended working channel 80 represent the change in location between the location to which the distal end 88 of the extended working channel 80 was navigated to, and the position of the distal end 88 following insertion of surgical instruments 102-108. It is contemplated that by using materials of known durometer ratings, the deflection of distal portion 84 of the extended working channel 80 can be accounted for such that the location of the extended working channel 80 at the end of the navigation phase translates to the location of the target tissue 68 after the deflection of distal portion 84 by the insertion of surgical instruments 102-108.

In embodiments, the durometer rating of the distal portion 84 is such that its deflection is limited. In this embodiment, positioning surgical instruments 102-108 within lumen 83 of the extended working channel 80 and extending surgical instruments 102-108 past the distal end 88 to treat the target tissue 68 will not significantly alter the radius of curvature "r" (e.g., the 45 degree radius of curvature "r") of the distal portion 84. Thus, there will not be a significant distance between the location of the extended working channel 80 at the end of the navigation phase and the target tissue 68. In another embodiment, the durometer rating of distal portion 84 is known to the user such that the deflection of distal portion 84 following the positioning of surgical instruments 102-108 within the extended working channel 80 can be accounted for during the navigation phase. As such, the location of extended working channel 80 at the end of the navigation phase translates to the location of the target tissue 68 after the deflection of distal portion 84 caused by the insertion of surgical instruments 102-108.

Referring back to Fig. 1, ENB system 10 includes tracking system 50 that is utilized for performing image to patient location registration and luminal navigation during the navigation phase. In embodiments, tracking system 50 is a six degrees-of-freedom electromagnetic tracking system similar to those disclosed in U.S. Patent No. 6,188,355 and published PCT Application Nos. WO 00/10456 and WO 01/67035, the entire contents of each of which are incorporated herein by reference. However, it is contemplated that other suitable positioning measuring systems as well as other suitable configurations may also be utilized. As depicted in Fig. 1, tracking system 50 includes tracking module 52, the plurality of reference sensors 54, and transmitter mat 56. Transmitter mat 56 of tracking system 50 is positioned beneath patient "P" and generates an electromagnetic field (not explicitly shown) around at least a portion of patient "P". Tracking system 50 is configured for use with catheter guide assembly 70.

Catheter guide assembly 70 includes control handle 90, a grip 92, and a telescopic shaft 94, which are operably connected to extended working channel 80. By rotating grip 92 and translating the telescopic shaft 94, the user is able to steer the extended working channel 80 to the target tissue 68 using one hand. These movements of control handle 90 enable the user to navigate the extended working channel 80 through the tortuous path of a luminal network such as the patient "P's" airways, and direct advancement of the extended working channel 80 at each bifurcation. An example of control handle 90 is currently manufactured and sold under the name EDGE™ by Covidien LP. Control handle 90 may be ergonomically shaped to facilitate grasping and/or rotation of extended working channel 80. In one embodiment, a plurality of rib portions (not explicitly shown) are provided along a length of control handle 90 to facilitate grasping and rotation of the extended working channel 80.

Using tracking module 52 and the generated electromagnetic field of transmitter mat 56, the position of the plurality of reference sensors 54 and sensor 74 may be determined as locatable guide 72 and extended working channel 80 are navigated through the luminal network. More particularly, one or more of the plurality of reference sensors 54 are attached to the chest of the patient "P". The coordinates of the plurality of reference sensors 54 and sensor 74 on the locatable guide 72 within the electromagnetic field generated by transmitter mat 56 are sent to computer 60, which includes the appropriate software to determine their location with reference to a navigation plan, as will be described in greater detail below.

The locatable guide 72 and the extended working channel 80 are configured for insertion through a working channel (not explicitly shown) of the bronchoscope 30 into the patient "P's" airways. However, it is contemplated that locatable guide 72 and extended working channel 80 may alternatively be used without the bronchoscope 30. In embodiments, the locatable guide 72 and the extended working channel 80 are selectively lockable relative to one another via a locking mechanism (not explicitly shown).

Having described the components of the ENB system 10 generally, the following describes their interaction and implementation in combination with planning and navigation software resident on computer 60. An example of such software for planning and navigation is the iLogic planning and navigation suites of software currently sold by Covidien LP.

During a planning phase, computer 60 utilizes computed tomographic (CT) image data for generating and viewing a three-dimensional model of patient "P's" airways. This process enables the identification of target tissue 68 to be navigated to in the image data and to coordinate that target with a three-dimensional model (automatically, semi-automatically, or manually). The planning software allows for the selection of a pathway through the patient "P's" airways to target tissue 68. More specifically, the CT scans are processed and assembled into a three-dimensional CT volume, which is then utilized to generate a three-dimensional model of the patient "P's" airways. The three-dimensional model of the patient "P's" airways may be displayed on a display associated with computer 60, or in any other suitable fashion. Using the computer 60, various views of the three-dimensional model of the patient "P's" airways or two-dimensional images generated from the three-dimensional model of the patient "P's" airways may be presented. The three-dimensional model of the patient "P's" airways or the two-dimensional images of the patient "P's" airways may be manipulated to facilitate identification of target tissue 68. Thereafter, a suitable pathway through patient "P's" airways to access target tissue 68 can be selected. Once a suitable pathway is selected, the pathway is saved and exported to a navigation component of the software for use during the navigation phase(s).

As an initial step, prior to insertion of the extended working channel 80 into bronchoscope 30, the user is able to choose the extended working channel 80 with the radius of curvature "r" best configured to reach target tissue 68. Following this selection but prior to beginning the procedure, the location of patient "P" on transmitter mat 56 must be registered. During registration, the location of target tissue 68 generated during the planning phase from the three-dimensional model of patient "P's" airways and the two-dimensional images of the patient "P's" airways is coordinated with the patient "P's" airways as observed through bronchoscope 30 and sensed using the sensor 74 when catheter guide assembly 70 is inserted into the lungs. As a result, the navigation phase can be undertaken with precise knowledge of the location of sensor 74 particularly in portions of the patient "P's" airways where bronchoscope 30 cannot reach. Further details of such a registration technique and its implementation in luminal navigation can be found in U.S. Patent Application Pub. No. 2011/0085720, the entire content of which is incorporated herein by reference. However, in embodiments, other suitable techniques are also contemplated.

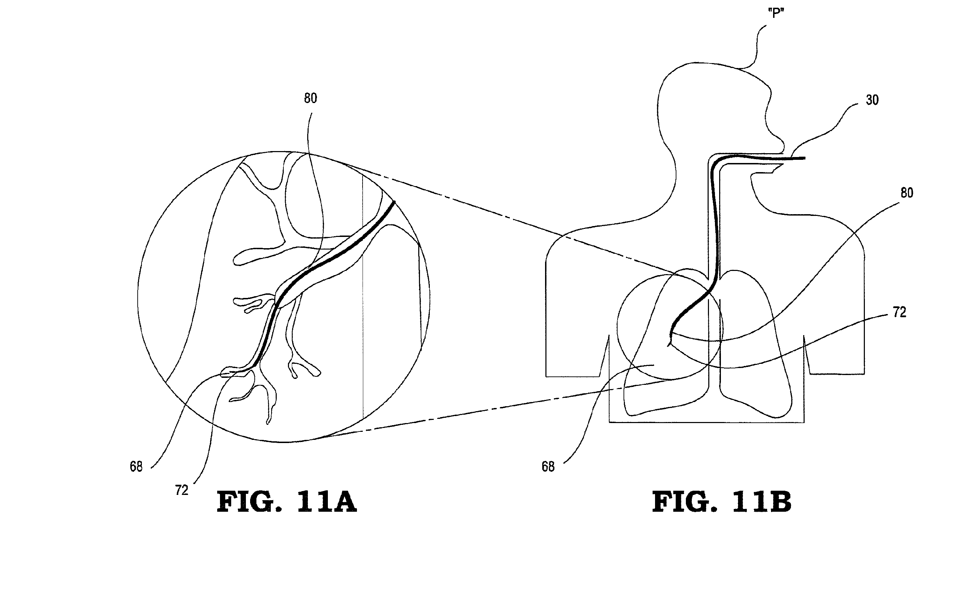

As described, sensor 74 is integrated into distal end 73 of locatable guide 72 and is configured to output signals that indicate the position and orientation of locatable guide 72 in six degrees of freedom, relative to a reference coordinate system. In use, locatable guide 72 is inserted into the extended working channel 80 such that sensor 74 projects from distal end 88 of the extended working channel 80. As mentioned above, locatable guide 72 and extended working channel 80 may be locked together via a locking mechanism (not explicitly shown). In embodiments, as shown in Figs. 1 and 11A and 11B, locatable guide 72, together with extended working channel 80, are inserted through bronchoscope 30 and into the airways of the patient "P". As a result, locatable guide 72 and extended working channel 80 move in concert through bronchoscope 30 and into the airways of patient "P". Initially, bronchoscope 30, including extended working channel 80 and locatable guide 72, are advanced through patient "P's" mouth and into the luminal network of patient "P". When bronchoscope 30 is wedged and unable to advance any further through the luminal network of patient "P", the extended working channel 80 and locatable guide 72 are advanced further without bronchoscope 30. As discussed in detail above, extended working channel 80 and locatable guide 72 are advanced to target tissue 68 by translating and rotating control handle 90.

In one embodiment automatic registration of the location of sensor 74 is undertaken by simply moving locatable guide 72 through the airways of the patient "P". More specifically, while locatable guide 72 is moving through the airways of the patient "P", data pertaining to the locations of sensor 74 are recorded using tracking system 50. A shape resulting from this location and movement data is compared with an interior geometry of airways of patient "P" from the three-dimensional model generated during the planning phase. Based on this comparison, a location correlation between the shape and the three-dimensional model of patient "P's" airways is determined, e.g., utilizing software on computer 60. The software on computer 60 aligns, or registers, an image representing a location of sensor 74, with the three-dimensional model of the airways of the patient "P" or the two-dimensional images generated from the three-dimension model, which are based on the recorded location data and an assumption that locatable guide 72 remains located in non-tissue space (e.g., within the air filled cavities) in the airways of patient "P".

After the planning phase has been completed (e.g., target tissue 68 has been identified and the pathway thereto selected), and the registration has been completed, ENB system 10 may be utilized to navigate locatable guide 72 through patient "P's" airway to target tissue 68. To facilitate such navigation, computer 60, monitoring equipment 40, and/or any other suitable display may be configured to display the three-dimensional model of the patient "P's" airways or two-dimensional images generated from the three-dimensional model of the patient "P's" airways as well as live images from bronchoscope 30. It is contemplated that both the three-dimensional model of patient "P's" airways and the two-dimensional images may include the selected pathway from the current location of sensor 74 to target tissue 68.

Once locatable guide 72 has been successfully navigated to target tissue 68, thus completing the navigation phase, locatable guide 72 may be unlocked from extended working channel 80 and removed, leaving the extended working channel 80 in place as a guide channel for guiding surgical instruments, such as, for example, biopsy forceps 102, cytology brush 104, aspirating needle 106, and ablation catheter 108 to target tissue 68. Prior to removal of locatable guide 72 or following insertion of surgical instruments 102-108, placement may be confirmed (e.g. within and/or adjacent the target tissue 68) utilizing one or more imaging modalities. For example, CT, ultrasound, fluoroscopy, and other imaging modalities may be utilized individually or in combination with one another.

From the foregoing and with reference to the various figure drawings, those skilled in the art will appreciate that certain modifications can also be made to the present disclosure without departing from the scope of the same. For example, distal portion 84 of extended working channel 80 may be provided with a closed distal end 88 instead of the open distal end 88 described above. This embodiment has particular use when the extended working channel 80 is configured to circulate a cooling fluid in a closed loop scheme for cooling an ablation catheter (not specifically shown), which may be positioned at distal end 88 to ablate target tissue 68.

While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope and spirit of the claims appended hereto.

## Claims

1. A catheter guide assembly, comprising:
an extended working channel including a proximal portion adjacent a proximal end and a distal portion adjacent a distal end, the extended working channel having a lumen configured to enable translation of an instrument therethrough, the extended working channel defining a radius formed on the distal portion, wherein the distal portion of the extended working channel is formed of a material having a durometer rating sufficient such that deflection of the distal portion is limited as an instrument is translated therethrough;
a control handle disposed at the proximal end of the extended working channel and operably coupled to the extended working channel, thereby enabling the extended working channel to advance and rotate.

2. The catheter guide assembly according to claim 1, wherein the extended working channel is configured for positioning within a bronchoscope.

3. The catheter guide assembly according to claim 1 or 2, wherein a locatable guide including at least one sensor is positionable within the lumen such that the location of the locatable guide within a luminal network can be detected.

4. A system for accessing a target within a luminal network, the system comprising:
a surgical instrument;
an extended working channel including a proximal portion adjacent a proximal end and distal portion adjacent a distal end, the extended working channel having a lumen configured to enable translation of the instrument therein, the extended working channel defining a radius formed on the distal portion, wherein the distal portion of the extended working channel is formed of a material having a durometer rating sufficient such that deflection of the distal portion is limited as the instrument is passed therethrough;
a control handle disposed at the proximal end of the extended working channel and operably coupled to the proximal end of the extended working channel, thereby enabling the extended working channel to advance and rotate;
a locatable guide insertable through the extended working channel;
a non-transitory computer readable medium storing a program for causing a computer to determine a pathway through a luminal network from an entry point to a target tissue and assign a plurality of waypoints along the pathway; and
a user interface configured to display an orientation of the locatable guide relative to the determined pathway as the extended working channel and the locatable guide are translated through the luminal network from the entry point towards the target tissue.

5. The system according to claim 4, wherein the locatable guide includes an at least one locatable guide sensor positionable within the lumen of the extended working channel, wherein the at least one locatable guide sensor is configured to enable detection of a location of the locatable guide within the luminal network of a patient's airways.

6. The system according to claim 5, wherein the at least one locatable guide sensor is disposed within the lumen of the extended working channel at a distal end thereof.

7. The system according to claim 4, 5 or 6, wherein the surgical instrument is selected from a group consisting of a biopsy forceps, a cytology brush, an ablation catheter, and an aspirating needle.

8. The system according to claim 4, 5, 6 or 7, further comprising a tracking system including a tracking module, a plurality of reference sensors, and a transmitter mat, at least one of the plurality of reference sensors being attached to a patient, wherein the transmitter mat generates an electromagnetic field such that the tracking module may be used to determine a location of the at least one of the plurality of reference sensors and the locatable guide sensor.

9. The system according to any one of claims 4 to 8, wherein the proximal portion of the extended working channel comprises a first durometer rating and the distal portion of the extended working channel comprises a second durometer rating, the first durometer rating is greater than the second durometer rating; or
the catheter guide assembly according to claim 1, 2 or 3, wherein the proximal portion of the extended working channel comprises a first durometer rating and the distal portion of the extended working channel comprises a second durometer rating, the first durometer rating is greater than the second durometer rating.

10. The system according to claim 9, wherein the distal end comprises the first durometer rating; or
the catheter guide assembly according to claim 9, wherein the distal end comprises the first durometer rating.

11. The system according to claim 9 or 10, wherein the first durometer rating ranges from about 30D to about 80D and the second durometer rating ranges from about 30D to about 63D; or
the catheter guide assembly according to claim 9 or 10, wherein the first durometer rating ranges from about 30D to about 80D and the second durometer rating ranges from about 30D to about 63D.

12. The system according to any one of claims 4 to 11, wherein the durometer rating of the distal end is different from the durometer rating of the distal portion; or
the catheter guide assembly according to any one of claims 1 to 3 or 9 to 11 claim 4, wherein the durometer rating of the distal end is different from the durometer rating of the distal portion.

13. The system according to any one of claims 4 to 12, wherein the distal portion comprises a length that ranges from about 5 percent to about 15 percent of an overall length of the extended working channel; or
the catheter guide assembly according to any one of claims 1 to 3 or 9 to 12, wherein the distal portion comprises a length that ranges from about 5 percent to about 15 percent of an overall length of the extended working channel.
